Europäisches Patentamt

European Patent Office

Office européen des brevets

(11) Veröffentlichungsnummer: **0 418 761 A2**

# (12) EUROPÄISCHE PATENTANMELDUNG

(21) Anmeldenummer: 90117779.0

(22) Anmeldetag: 15.09.90

(51) Int. Cl.⁵: **B25B 7/14, A61B 17/28**

(30) Priorität: 22.09.89 DE 3931577

(43) Veröffentlichungstag der Anmeldung:
27.03.91 Patentblatt 91/13

(84) Benannte Vertragsstaaten:
**BE DE FR GB IT**

(71) Anmelder: **Richard Wolf GmbH**
**Pforzheimer Strasse 32**
**W-7134 Knittlingen(DE)**

(72) Erfinder: **Boebel, Manfred**
**Allmandstrasse 18**
**W-7136 Oetisheim(DE)**

(74) Vertreter: **Wilcken, Thomas, Dipl.-Ing. et al**
**Musterbahn 1**
**W-2400 Lübeck(DE)**

(54) Feststellvorrichtung zwischen den Handgriffarmen medizinischer Zangen.

(57) Die beschriebene Feststellvorrichtung an einer medizinischen Zange zum Festhalten von Gewebeteilen oder Fremdteilen während eines medizinischen Eingriffes besteht aus zwei länglichen Federelementen (1,2), deren abgekehrte Enden in geschlossenen Teleskoprohrenden (3,4) befestigt sind. Die Teleskoprohre (3,4) sind gelenkig mit Handgriffen (6,7) einer Zange verbunden. Durch Zusammendrücken der teilweise elastisch nachgiebigen Handgriffe (6,7) werden die Federelemente (1,2) durch Gegeneinanderverschieben mittels Auflaufflächen (10,11) hakenförmig in Eingriff gebracht, womit das durch die Zange erfaßte Gewebe oder dergleichen nach Aufheben des Handdrucks auf die Handgriffe (6,7) automatisch festgehalten wird. Durch weiteres Zusammendrücken der Handgriffe (6,7) wird der Hakeneingriff aufgehoben, so daß sich die Handgriffe und die Zangenmaulbacken zur Freigabe des erfaßten Gewebes oder dergleichen durch Abfederung der Handgriffe (6,7) öffen.

Fig.1

# FESTSTELLVORRICHTUNG ZWISCHEN DEN HANDGRIFFARMEN MEDIZINISCHER ZANGEN

Die Erfindung geht aus von einer Feststellvorrichtung, die zwischen den Handgriffen einer Zange angeordnet ist, insbesondere für ein medizinisches Instrument, bestehend aus einer zwischen den Zangenhandgriffen angeordneten Spreizfeder, zwei mit den Enden der Zangenhandgriffe verbundenen Halte- und Führungsteilen und einer mit Federwirkung arbeitenden Verriegelungseinrichtung.

In der medizinischen Technik, insbesondere in der Endoskopie, werden Instrumente und Zangen eingesetzt, um Gewebeteile oder auch andere Teile erfassen und behandeln oder entfernen zu können. Dabei ist es wichtig, daß das Festhalten der erfaßten Teile durch das Instrument während eines medizinischen Eingriffes gewährleistet ist. Zu diesem Zweck sind Feststellvorrichtungen für gegen eine Abfederung zusammendrückbare Handgriffe medizinischer Instrumente bzw. Zangen bekannt, wie sie in den US-Patentschriften 4 644 651 und 4 572 185, den DE-Patentschriften 32 23 513 und 836 545 sowie dem DE-Gebrauchsmuster 83 16 034 beschrieben sind.

Der Erfindung liegt die Aufgabe zugrunde, die Feststellvorrichtung so auszubilden, daß die Handgriffe medizinischer Instrumente bzw. einer endoskopischen Faßzange mit einer Hand zusammengedrückt und anschließend wieder durch die Hand gelöst werden können. Dabei soll die Feststellvorrichtung gegen Beschädigungen geschützt sein.

Diese Aufgabe wird durch die Merkmale des Anspruches 1 gelöst. Eine bevorzugte Ausgestaltung ist durch den Anspruch 2 gekennzeichnet.

Durch die Lösung nach der Erfindung kann der Benutzer die Handgriffe mit nur einer Hand zusammendrücken; dabei wird die zusammengedrückte Lage automatisch nach Überwindung eines ersten Druckpunktes unter sicherem Festhalten von Gewebe im Zangenmaul fixiert. Durch weiteres Zusammendrücken der Handgriffe und Überwindung eines zweiten Druckpunktes wird die Fixierung aufgehoben und die Handgriffe können durch ihre Abfederung in ihre gespreizte Lage zurückkehren. Dabei ist die Feststellvorrichtung innerhalb der Teleskoprohre gegen Beschädigungen geschützt, so daß durch die Feststellvorrichtung Verletzungen des Benutzers nicht mehr auftreten.

Die Erfindung ist nachstehend anhand der Zeichnung beispielsweise erläutert. Es zeigen:

Figur 1 die Feststellvorrichtung aus den beiden Blattfedern in zwei zueinander senkrechten Ansichten der Teile mit Schnitt durch das die Feststellvorrichtung aufnehmende Teleskop,

Figur 2 die beiden Darstellungen nach Figur 1 nach Erreichen des Eingriffs der beiden Blattfedern,

Figur 3 die Stellung der Feststellvorrichtung, in der die beiden Blattfedern außer Eingriff gelangen.

Die Feststellvorrichtung für das Festhalten einer geschlossenen Stellung eines Zangenmaules oder dergleichen besteht aus zwei Blattfedern 1 und 2, die an ihrem einen Ende in Teleskoprohren 3 und 4 befestigt sind, welche durch ein teleskopisches Zwischenrohr 5 verbunden sind. Die Rohre 3 und 4 sind mit ihren voneinander abgekehrten, geschlossenen Enden gelenkig mit den beiden elastischen nachgiebigen Handgriffarmen 6 und 7 einer Zange oder dergleichen verbunden.

Die eine Verriegelungseinrichtung bildenden beiden Blattfedern besitzen in der gespreizten Stellung der beiden Handgriffe 6, 7 einen Abstand voneinander. Dabei weist die obere Blattfeder 1 am freien Ende einen einseitig aus der Blattfederebene herausragenden Haken $11'$ mit einer rückwärts geneigten vorderen Auflauffläche 11 und mit einer rückwärts gerichteten hinteren Hakenfläche auf. Die untere Blattfeder 2 ist an ihrem freien Ende mit einem in gleicher Richtung wie der Haken $11'$ aus der Blattfederebene herausragenden Vorsprung $10'$ versehen, der eine vorwärtsgeneigte vordere Auflauffläche 10 und eine gleichfalls vorwärtsgeneigte hintere Auflautfläche $10''$ aufweist. Im Abstand vom Haken $11'$ und dem Vorsprung $10'$ ist in den Blattfedern 1 und 2 spiegelbildlich zueinander ein längsgerichteter, seitlicher Ausschnitt 1a und 2a angeordnet bzw. sind die Blattfedern 1, 2 an ihrem freien, einander zugekehrten Enden je mit einer Kopfverbreiterung 8 und 9 versehen, die in Querrichtung entgegengesetzt gerichtet sind und sich gegenüberliegen.

Beim Zusammendrücken der Handgriffe 6, 7 gegen ihre Abfederung gleiten die Auflaufflächen 10 und 11 unter seitlichem Ausweichen der Blattfedern aneinander entlang und es entsteht ein erster Druckpunkt beim Übergreifen des Vorsprunges $11'$, wobei die Hakenfläche 12 über eine Auflauffläche 13 der unteren Kopfverbreiterung greift bzw. wobei der Haken 12 in die Ausnehmung 13 greift, wodurch die Verriegelungsstellung der Handgriffe 6,7 mit Festhalten von Gewebeteilen durch das Zangenmaul oder dergleichen erreicht ist. Soll diese Verriegelungslage des Zangenmaules aufgehoben werden, so werden die elastisch nachgiebigen Handgriffarme 6, 7 weiter zusammengedrückt, bis die Auflauffläche 13 der Blattfeder 2 eine Lage hinter der Auflauffläche 14 bzw. der Kante des Ausschnittes 1a der Blattfeder 1 einnimmt. Werden nun die Handgriffe 6, 7 losgelassen, so werden sie durch ihre Abfederung gespreizt, und dadurch werden die Blattfedern unter Auseinanderziehen der Teleskoprohre 3, 4, 5 auseinandergezogen, wobei sich die beiden Blattfedern in Querrichtung zu ihrer Ebene durch die aneinander gleitenden Auflaufflächen 13, 14 bewegen und voneinander frei werden.

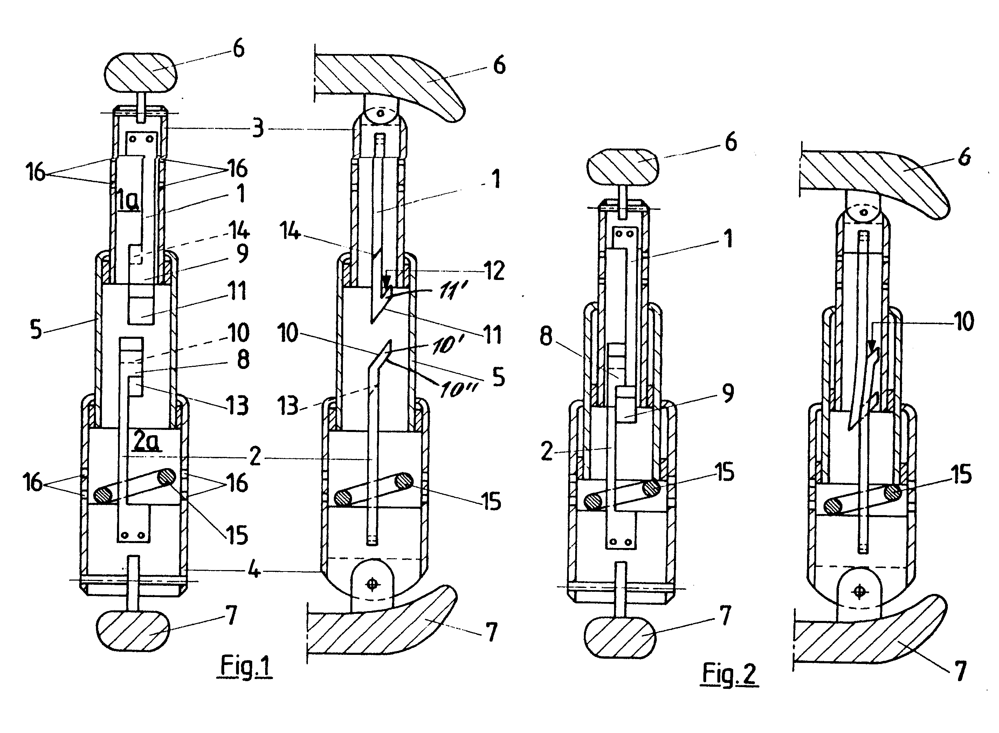

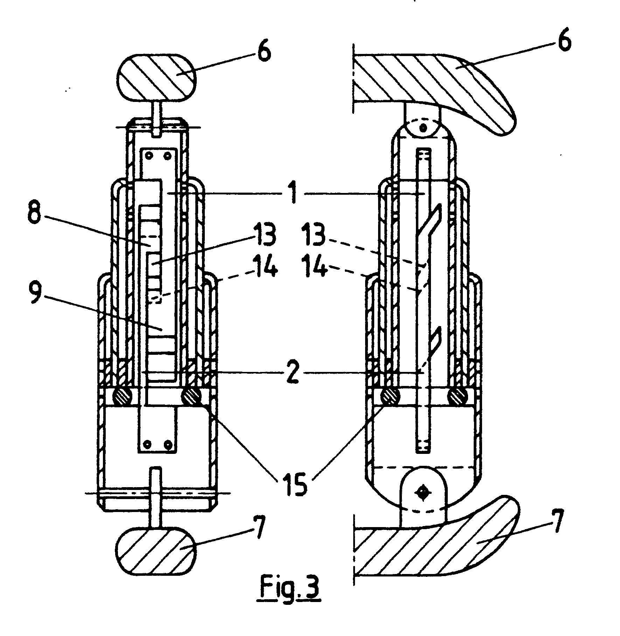

Die Verriegelungsstellung ist in Figur 2, die Stellung zum Lösen der im Handgriff befindlichen Blattfedern in Figur 3 gezeigt. Damit die vorerwähnten Vorgänge durchgeführt werden können, ist die Kopfverbreiterung 9 der Blattfeder 1 in der Längsrichtung größer gewählt als die längsgerichtete Länge der Kopfverbreiterung 8 der Blattfeder 2.

Beim Gegeneinanderschieben der Blattfedern 1, 2 aus der Stellung Figur 1 in die Stellung nach Figur 2 werden die Blattfedern 1,2 in dem Teleskoprohr 3 dadurch geführt, daß dieses einen Innendurchmesser aufweist, der der etwa eineinhalbfachen Breite eines Kopfes der Blattfedern entspricht.

Die Feststellvorrichtung ist weiter so ausgebildet, daß beim Lösen der Verriegelung ein zweiter Druckpunkt vorhanden ist, der mittels einer die Zangenarme spreizenden Feder 15 entsteht, und zwar durch Zusammendrücken der im oberen Teleskoprohr entgegenwirkenden Feder 15.

## Ansprüche

1. Feststellvorrichtung, die zwischen den Handgriffen einer Zange angeordnet ist, insbesondere für ein medizinisches Instrument, bestehend aus

1.1. einer zwischen den Zangenhandgriffen angeordneten Spreizfeder,

1.2. zwei mit den Enden der Zangenhandgriffe verbundenen Halte- und Führungsteilen,

1.3. einer mit Federwirkung arbeitenden Verriegelungseinrichtung,
dadurch gekennzeichnet, daß

1.4. die Halte- und Führungsteile aus mindestens zwei Teleskoprohren (3,4) bestehen,

1.5. die Verriegelungseinrichtung aus einer ersten Blattfeder (1) und einer zweiten Blattfeder (2) (nachfolgend obere und untere Blattfeder genannt) besteht, diese Blattfedern jeweils mit einem Ende am griffseitigen Ende innerhalb der Teleskoprohre (3,4) befestigt sind und zueinander in der gespreizten Stellung der Zangenhandgriffe im Bereich ihrer freien Enden einen Abstand aufweisen, wobei

1.5.1 die obere Blattfeder (1) am freien Ende einen einseitig aus der Blattfederebene herausragenden Haken (11') mit einer rückwärts geneigten vorderen Auflauffläche (11) und mit einer gleichfalls rückwärts geneigten hinteren Hakenfläche (12) aufweist sowie

1.5.2. die untere Blattfeder (2) an ihrem freien Ende mit einem in gleicher Richtung wie der Haken (11') aus der Blattfederebene herausragenden Vorsprung (10') versehen ist, der eine vorwärts geneigte vordere Auflauffläche (10) und eine gleichfalls vorwärts geneigte hintere Auflauffläche (10") aufweist,

1.6. schließlich jeweils im Abstand zum Haken (11') und dem Vorsprung (10') in den Blattfedern (1,2) spiegelbildlich zueinander ein langgestreckter Ausschnitt (1a,2a) angeordnet ist, wobei

1.6.1. nach dem Überwinden eines ersten Druckpunktes, der mitteils einer auf die Zangenhandgriffe wirkenden und die Blattfedern aufeinander zuschiebenden Kraft durch Auslenkung der unteren Blattfeder (2) beim Hakenübergreifen entsteht und beim gleichzeitigen Eintauchen des Hakens in den Ausschnitt (2a) eine vordere Ausschnittsfläche (13) den Haken in einer Verriegelungsstellung hintergreift

1.6.2. nach dem Überwinden eines zweiten Druckpunktes, der mittels einer weiteren auf die Zangenschenkel wirkenden und die Blattfedern weiter übereinander schiebenden Kraft durch Zusammendrücken einer dem oberen Teleskoprohr (3) im Wege stehenden Feder (15) entsteht, und daß hierbei der vor dem Ausschnitt (2a) der unteren Blattfeder (2) liegende Teil dieser Blattfeder einschließlich des Vorsprungs (10') fluchtend in den Ausschnitt (1a) der oberen Blattfeder (3) einzutauchen vermag und daß die untere Blattfeder (2) beim Spreizen der Zangenhandgriffe (6,7) durch entgegengesetztes Auslenken des Vorsprungs (10) an der vorderen Kante (14) des Ausschnittes (1a) der oberen Blattfeder (1) in die Ausgangsstellung zurückzukehren vermag.

2. Halteelement nach Anspruch 1, dadurch gekennzeichnet, daß im unteren Teleskoprohr (4) des Zangenhandgriffes (7) mindestens ein Federelement (15) für den zweiten Druckpunkt abgestützt ist, das von dem mit dem anderen Zangenhandgriff verbundenen Teleskoprohr (3) beaufschlagbar ist.

Fig.1

Fig.2

Fig. 3